# EUROPEAN PATENT SPECIFICATION

(11) **EP 1 289 942 B1**
(45) Date of publication and mention of the grant of the patent: **07.05.2008**
(21) Application number: 01902384.5
(22) Date of filing: 02.02.2001
(51) Int. Cl.: C07C 273/04

(54) **PROCESS AND PLANT FOR THE PRODUCTION OF UREA**
VERFAHREN UND VORRICHTUNG ZUR HERSTELLUNG VON HARNSTOFF
PROCEDE DE PRODUCTION D'UREE, ET INSTALLATION CORRESPONDANTE

(30) Priority: 15.06.2000 EP 00112688
(43) Date of publication of application: 12.03.2003
(73) Proprietor: UREA CASALE S.A., 6900 Lugano-Besso (CH)
(72) Inventor: PAGANI, Giorgio, CH-6900 Lugano (CH); ZARDI, Federico, CH-6932 Breganzona (CH); ROMITI, Domenico, CH-6900 Lugano (CH)
(74) Representative: Zardi, Marco
(86) International application number: PCT/EP2001/001098
(87) International publication number: WO 2001/096287

(56) References cited:
- WO-A-00/00466
- WO-A-96/23767

## Description

### Technical Field

In its most general aspect the present invention relates to a process for urea production.

Specifically, the present invention relates to a process for urea production of the type comprising the steps of:
- performing a reaction between ammonia and carbon dioxide in a reaction space to obtain a reaction mixture comprising urea, carbamate and free ammonia in aqueous solution,
- subjecting said mixture to a stripping treatment with the use also of carbon dioxide feed as a stripping agent to obtain a first flow comprising ammonia and carbon dioxide in vapor phase and a flow comprising urea and residual carbamate in aqueous solution,
- subjecting said first flow comprising ammonia and carbon dioxide in vapor phase to a substantially total condensation to obtain a first portion of carbamate in aqueous solution,
- recycling said first portion of carbamate in aqueous solution to said reaction space,
- feeding said flow comprising urea and residual carbamate in aqueous solution to a urea recovery section,
- separating in said recovery section said residual carbamate from the urea to obtain a second portion of carbamate in aqueous solution.

The present invention also relates to a plant for carrying out the above mentioned process and a method for modernizing a urea production plant according to said process.

As known, with respect to the urea production, the need is ever more growing of plants having greater capacity and operating flexibility on the one hand and on the other hand, requiring ever smaller investment and operating costs.

### Background Art

It is well known to produce urea by a process as specified above, which is carried out in a urea plant based on the so called "carbon dioxide stripping technology".

Such a plant is characterized by a substantially isobaric loop comprising as main components a synthesis reactor or reaction space, a stripper and a carbamate condenser.

According to the stripping technology, the majority of the unconverted ammonium carbamate is decomposed and the majority of the excess ammonia is removed at pressures nearly the same as the pressure in the synthesis reactor.

This decomposition and removal occurs in the stripper installed downstream of the synthesis reactor. Although thermal stripping alone may be used, more typically, the reaction mixture comprising urea, carbamate and free ammonia in aqueous solution is fed into the stripper and a stripping gas, generally carbon dioxide, is also used to decompose the ammonium carbamate and remove the majority of the carbon dioxide and ammonia from the solution.

The gas stream coming from the stripper comprises mainly ammonia and carbon dioxide and is typically fed into a carbamate condenser operating at or near the synthesis pressure to produce an ammonium carbamate solution that can be fed back into the synthesis reactor.

A configuration for a urea production plant, which employs the stripping process with carbon dioxide is referred to as the DSM carbon dioxide stripping process and is described in WO 00/00466.

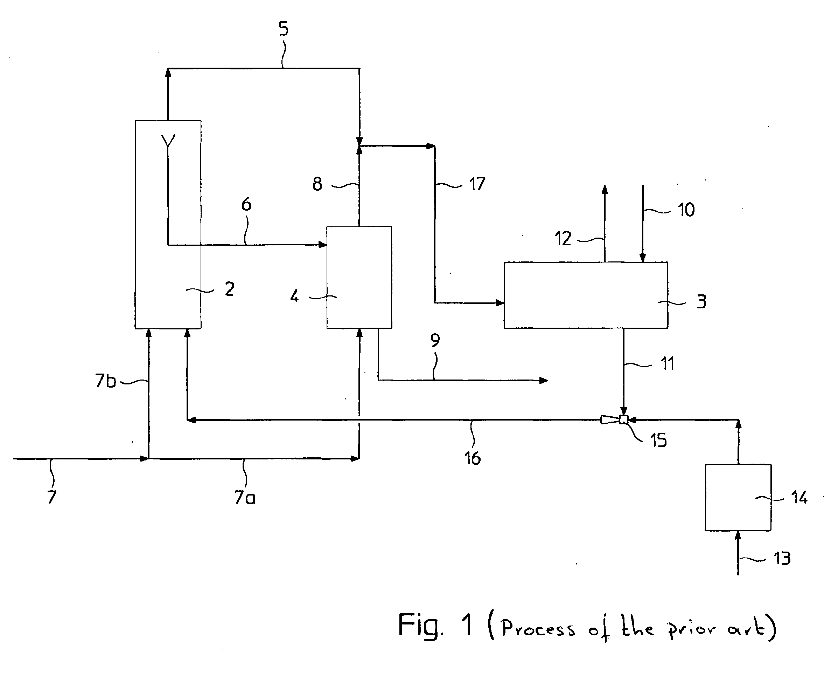

In this process, illustrated schematically in the annexed figure 1, the vapor stream coming from the stripper is totally condensed in a carbamate submerged condenser and an ejector with liquid ammonia as driving fluid is used for recycling to the reactor the carbamate aqueous solution leaving the carbamate condenser.

In this way, it is possible to place all of the apparatus of the isobaric loop namely, the synthesis reactor, condenser and the stripper at the ground level (horizontal layout) reducing the investment costs and making easier the construction, maintenance and operation of the plant.

However, according to the above process, a substantial portion of the carbon dioxide feed needs to be directly sent to the reactor to secure the reactor thermal balance, instead of being first sent to the stripper as stripping gas.

This significantly reduces the stripping efficiency leaving higher residual content of unreacted ammonia and carbon dioxide in the stripped urea solution to be processed in the urea recovery section with ensuing higher investment and operating costs.

In order to reduce the above drawback, it has been proposed to produce most of urea in the condenser adapting the latter to be a "condenser-reactor" and consequently reducing the amount of the carbon dioxide feed to be sent to the reactor to secure the thermal balance.

However, a condenser-reactor is an apparatus technically complicated to be manufactured and operated and involves higher operation and maintenance costs than a conventional condenser.

Furthermore, although according to the process of WO 00/00466 most of urea is formed in the condenser (see examples), suggesting that a condenser-reactor is employed, a substantial part of the carbon dioxide feed (about 21%) is still sent directly to the reactor.

A similar configuration for a urea production plant is also disclosed in US 5,936,122 (Toyo process), which suggests the possibility of sending part of the carbon feed directly to the reactor and, at the same time, to produce the majority of the urea in a high-pressure condenser-reactor.

A process and plant for urea production is also disclosed in WO 96/23767.

### Disclosure of Invention

The technical problem underlying the present invention is that of providing a process for urea production, which achieves a high conversion yield and, at the same time, would be technically simple to implement and would involve low investment, maintenance and operating costs.

In accordance with the present invention, this problem is solved by a process of the above mentioned type, which is characterized in that it comprises the additional steps of:
- subjecting at least part of said second portion of carbamate in aqueous solution obtained in said urea recovery section to a treatment of partial decomposition to obtain a second flow comprising ammonia and carbon dioxide in vapor phase and a flow comprising residual carbamate in aqueous solution,
- recycling at least part of said second flow comprising ammonia and carbon dioxide in vapor phase to said reaction space and/or using at least part of said second flow comprising ammonia and carbon dioxide in vapor phase for the stripping treatment of said reaction mixture comprising urea, carbamate and free ammonia in aqueous solution.

In order to obtain almost water free vapors and easy recycle to reactor of the same, said at least part of the second portion of carbamate in aqueous solution is preferably subjected to the treatment of partial decomposition at a pressure substantially corresponding to the pressure in the reaction space.

The process of the present invention comprises also the step of feeding the flow comprising residual carbamate in aqueous solution resulting from the treatment of partial decomposition of at least part of the second portion of carbamate to said urea recovery section.

According to the present invention, preferably at least 50% of the carbamate in aqueous solution leaving the urea recovery section, most preferably from 70% to 100%, is advantageously subjected to a treatment of partial decomposition separating unreacted ammonia and carbon dioxide from a solution rich in water comprising residual carbamate.

Furthermore, preferably at least 40% of said second flow comprising ammonia and carbon dioxide, most preferably from 90% to 100% is recycled to the reaction space.

So doing, it has been surprisingly found that if the above unreacted substances are recycled to the reactor space, the portion of carbon dioxide feed to be used for the stripping treatment of the reaction mixture can be highly increased or even all carbon dioxide feed can be used for said stripping treatment without the need of sending substantial portions of it directly to the reactor or producing most of the urea in a condenser-reactor as in the process according to WO 00/00466 or the process according to US 5,936,122.

In this way, the above mentioned unreacted substances, i.e. carbon dioxide and ammonia in vapor phase and possibly the low portion of the carbon dioxide feed directly sent to the reactor secure the heat balance of the reaction space and reduce the operating costs with respect to the prior art.

Particularly, it has been surprisingly found that at least 90% of the carbon dioxide feed can be used in the stripping treatment of the reaction mixture with consequent optimal stripping efficiency.

By operating in this manner, it is also possible to obtain a high conversion yield in the reaction space since, by virtue of the high efficiency of the stripping treatment of the reaction mixture and of the decomposition of at least part of the carbamate solution coming from the urea recovery section achieved according to the invention, a high recover of unreacted substances within the high-pressure loop is obtained while, at the same time, a highly concentrated solution of carbamate, which is very poor in water, is recycled - in vapor phase - to said reaction space.

Moreover, the substantially total condensation of the ammonia and carbon dioxide vapors coming from the stripping treatment of the reaction mixture allows the use of suitable means, such as an ejector driven by at least part of the ammonia feed, for recycling the carbamate solution to the reaction space.

In this way, it is advantageously possible to place all of the synthesis loop (reactor, carbamate condenser and carbamate strippers) at the ground level reducing the investment costs and making easier the construction, maintenance and operation of the plant.

The above-mentioned total condensation also allows the separation of the main part of inert gases (mainly air introduced through the carbon dioxide feed to protect equipment against corrosion) contained in the ammonia and carbon dioxide vapors coming from the stripping treatment of the reaction mixture and thus minimize the content of inert gases in the reaction space with consequent better carbon dioxide conversion efficiency.

According to another aspect of the present invention, the technical problem set forth above is solved by a plant designed to implement the above mentioned urea production process comprising:
- a urea synthesis reactor for performing a reaction between ammonia and carbon dioxide obtaining a reaction mixture comprising urea, carbamate and free ammonia in aqueous solution,
- a first thermal stripping unit with also carbon dioxide as stripping agent for subjecting said reaction mixture to a treatment of partial decomposition of the carbamate and partial separation of the free ammonia in aqueous solution present in said mixture obtaining a first flow comprising ammonia and carbon dioxide in vapor phase and a flow comprising urea and residual carbamate in aqueous solution,
- means for substantially totally condensing said first flow comprising ammonia and carbon dioxide in vapor phase obtaining a first portion of carbamate in aqueous solution,
- means for recycling said first portion of carbamate in aqueous solution to said reactor,
- a urea recovery section for separating urea from said flow comprising urea and residual carbamate in aqueous solution leaving the first stripping unit, obtaining a second portion of carbamate in aqueous solution,
which plant is characterized in that it comprises:
- a second stripping unit for subjecting at least part of said second portion of carbamate in aqueous solution to a treatment of partial decomposition obtaining a second flow comprising ammonia and carbon dioxide in vapor phase,
- means for recycling at least part of said second flow comprising ammonia and carbon dioxide in vapor phase to the urea synthesis reactor and/ or to the first stripping unit.

Preferably, the said means for substantially totally condensing the first gaseous flow leaving the first stripping unit comprise a carbamate condenser of the "submerged type", i.e. a known apparatus wherein the liquid phase fills (submerges) a tube bundle and wherein the condensation of the gaseous phase occurs by passing the same through such liquid phase.

According to an aspect of the present invention, the above plant further comprises means for feeding a first portion of liquid ammonia feed in said reactor for urea synthesis and means for feeding a second portion of liquid ammonia feed to said carbamate condenser.

Preferably, the amount of liquid ammonia to be sent to the reactor (first portion) is more than the amount of liquid ammonia to be sent to the carbamate condenser (second portion).

The feed of a second portion of ammonia in the carbamate condenser significantly promotes the total condensation of the vapors coming from the first stripping unit.

According to another aspect of the present invention, the above plant further comprises:
- means for preheating said at least part of the second portion of carbamate solution to be sent to the second stripping unit.

Additionally, the above plant further comprises:
- means for preheating said first portion of liquid ammonia feed to be sent to the urea synthesis reactor and/or said second portion of liquid ammonia feed to be sent to the carbamate condenser.

Advantageously, the above preheating is achieved using the heat removed in the carbamate condenser to secure minimum energy consumption.

According to the present invention, the means for feeding the first portion of liquid ammonia feed to the reactor and the means for recycling the first portion of carbamate in aqueous solution to the reactor comprise:
- an ejector,
- means for feeding said first portion of carbamate in aqueous solution to the ejector,
- means for feeding said first portion of liquid ammonia feed to the ejector, and
- means for sending said first portion of carbamate in aqueous solution together with said first portion of liquid ammonia feed from the ejector to the reactor for urea synthesis.

The above ejector advantageously uses the first portion of liquid ammonia feed as driving fluid.

According to a preferred embodiment of the present invention, the second stripping unit is a thermal stripping unit and the plant further comprises means for feeding all carbon dioxide feed to said first stripping unit or means for feeding a major portion of carbon dioxide feed to said first stripping unit and means for feeding a remaining minor portion of carbon dioxide feed to said reactor for urea synthesis.

According to a further embodiment of the present invention, the second stripping unit is a thermal stripping unit with also carbon dioxide as stripping agent and the plant further comprises:
- means for feeding a major portion of carbon dioxide feed to said first stripping unit and means for feeding a remaining minor portion of carbon dioxide feed to said second stripping unit and/or to said reactor for urea synthesis.

With a plant as described above, the process according to the present invention comprises the features recited in dependent claim 15.

Preferably, the treatment of partial decomposition of said at least part of the second portion of carbamate in aqueous solution is carried out in the second stripping unit at a pressure substantially corresponding to the pressure in the reactor.

Moreover, the above process is characterized in that it further comprises the step of:
- feeding the flow comprising residual carbamate in aqueous solution resulting from the treatment of partial decomposition of the second portion of carbamate to the urea recovery section.

According to an aspect of the present invention, the above process further comprises the steps of:
- feeding a first portion of liquid ammonia in said reactor for urea synthesis, and
- feeding a second portion of liquid ammonia to said carbamate condenser.

According to a preferred embodiment of the present invention, the second stripping unit is a thermal stripping unit and the process further comprises the step of feeding all carbon dioxide feed to said first stripping unit.

According to another preferred embodiment of the present invention, the second stripping unit is a thermal stripping unit and the process further comprises the steps of:
- feeding a major portion of carbon dioxide feed to said first stripping unit, and
- feeding a remaining minor portion of carbon dioxide feed to said reactor for urea synthesis.

According to another embodiment of the present invention, the second stripping unit is a thermal stripping unit with also carbon dioxide as a stripping agent and the process further comprises the steps of:
- feeding a major portion of carbon dioxide feed to said first stripping unit,
- feeding a remaining minor portion of carbon dioxide feed to said second stripping unit and/or to said reactor for urea synthesis.

In accordance with the present invention the plants for carrying out the urea production process may be either new or provided by modifying pre-existing plants. In the latter case, a production capacity expansion may be obtained and possibly a reduction of the energy consumption.

According to another aspect, the present invention accordingly makes available a method for modernizing a urea production plant of the type comprising:
- a reactor for urea synthesis;
- a first thermal stripping unit with also carbon dioxide as a stripping agent for subjecting a reaction mixture comprising urea, carbamate and free ammonia in aqueous solution leaving the reactor to a treatment of partial decomposition of carbamate and partial separation of carbamate and partial separation of free ammonia, thus obtaining a first flow comprising ammonia and carbon dioxide in vapor phase and a flow comprising urea and residual carbamate in aqueous solution, respectively,
- a urea recovery section for separating urea from said flow comprising urea and residual carbamate in aqueous solution leaving the first stripping unit, obtaining a second portion of carbamate in aqueous solution,
- at least one condensation unit for subjecting to partial condensation the first flow comprising ammonia and carbon dioxide in vapor phase leaving the first stripping unit, thus obtaining a liquid flow comprising carbamate in aqueous solution and a gaseous flow comprising ammonia and carbon dioxide in vapor phase,
- means for respectively feeding the flow comprising carbamate in aqueous solution and the gaseous flow comprising ammonia and carbon dioxide in vapor phase to the reactor for urea synthesis,
said modernization method being characterized in that it comprises the steps of:
- transforming said at least one condensation unit in a total condensation unit for subjecting to substantially total condensation said first flow comprising ammonia and carbon dioxide in vapor phase, obtaining a liquid flow comprising urea and carbamate in aqueous solution,
- providing a second stripping unit for subjecting at least part of said second portion of carbamate in aqueous solution to a treatment of partial decomposition, obtaining a second flow comprising ammonia and carbon dioxide in vapor phase,
- providing means for recycling at least part of the second flow comprising ammonia and carbon dioxide in vapor phase to the reactor and/or to the first stripping unit.

Further characteristics and advantages of the present invention are set forth in the detailed description of preferred embodiments thereof given below by way of non-limiting example with reference to the annexed drawings.

### Brief Description of Drawings

In such drawings:
- figure 1 illustrates schematically and partially a plant for urea production according to the stripping process of the prior art with also carbon dioxide as a stripping agent,
- figure 2 illustrates schematically and partially a plant for urea production according to the stripping process with also carbon dioxide as a stripping agent of the present invention,
- figure 3 illustrates schematically and partially another plant for urea production according to the stripping process with also carbon dioxide as a stripping agent of the prior art,
- figure 4 illustrates schematically and partially a plant for urea production according to the stripping process with also carbon dioxide as a stripping agent obtained by the modernization of the plant of figure 3 in accordance with a preferred embodiment of the method of modernization of the present invention.

### Modes for Carrying Out the Invention

Just to simplify the disclosure of the present invention, only a portion of a plant for urea production is schematically represented in the figures and more precisely the high-pressure synthesis section (synthesis loop), the remaining sections being not significant for the comprehension of the present invention.

Further on, specific reference to the per se conventional connecting ducts of the various parts of the plants described hereinbelow and illustrated in the figures, will be made only when strictly necessary.

With reference to figure 1, a DSM plant for urea production according to WO 00/00466, is indicated with reference numeral 1.

Plant 1, and more specifically the high-pressure synthesis loop comprises a reactor or reaction space 2 for urea synthesis, a condenser 3 and a stripping unit 4 with carbon dioxide.

Moreover, plant 1 comprises a recovery section for the urea produced, which is not represented in figure 1.

The condenser 3, which is of the "submerged type", and the stripping unit 4 generally operate at the same pressure conditions as the reaction space 2.

Flow line 6 represents a liquid flow of a reaction mixture coming from the reaction space 2 comprising urea, carbamate and free ammonia in aqueous solution.

This reaction mixture is fed into the stripping unit 4 where it is subjected to a treatment of partial decomposition of the carbamate and partial separation of said free ammonia in aqueous solution.

The stripping unit 4 operates with part of the carbon dioxide feed as stripping agent which is fed to the stripping unit 4 through the flow line 7a, the whole carbon dioxide feed being represented by the flow line 7.

The remaining part of the carbon dioxide feed is instead supplied to the reaction space 2 through the flow line 7b to secure the reactor thermal balance.

At the outlet of the stripping unit 4, flow lines 8 and 9 are shown which represent a gas flow comprising ammonia and carbon dioxide in vapor phase and a liquid flow comprising urea and residual carbamate in aqueous solution, respectively.

The liquid flow 9 is fed to the urea recovery section (not shown) where the urea is separated and a low-pressure carbamate in aqueous solution is obtained. This solution, indicated by flow line 10, is supplied to the condenser 3 as an absorption medium.

The gas flow 8 is also fed into the condenser 3 where the ammonia and carbon dioxide in vapor phase are brought into contact with the carbamate in aqueous solution coming from the urea recovery section promoting the condensation of said vapor phase.

By virtue of the above condensation, a flow comprising carbamate in aqueous solution and a substantial proportion of urea is obtained at the outlet of the condenser 3. Such a flow is represented by flow line 11.

At the outlet of the reactor 2, there is also shown a flow line 5 representing a flow of inert gases, which were previously introduced in the high-pressure synthesis section through the carbon dioxide feed.

The above flow of inert gases is combined with the gas flow comprising ammonia and carbon dioxide in vapor phase coming from the stripping unit 4 and the resulting flow, represented by the flow line 17, is fed to the condenser 3 where the inert gases are separated and vented from the condenser 3 through the flow line 12.

The ammonia feed represented by the flow line 13 is preheated in an heat exchanger 14 and sent to an ejector 15, which sucks up the liquid flow 11 coming from the condenser 3. The ammonia feed enters the ejector 15 as driving fluid and is sent together with the liquid flow 11 to the reaction space 2 through the flow line 16.

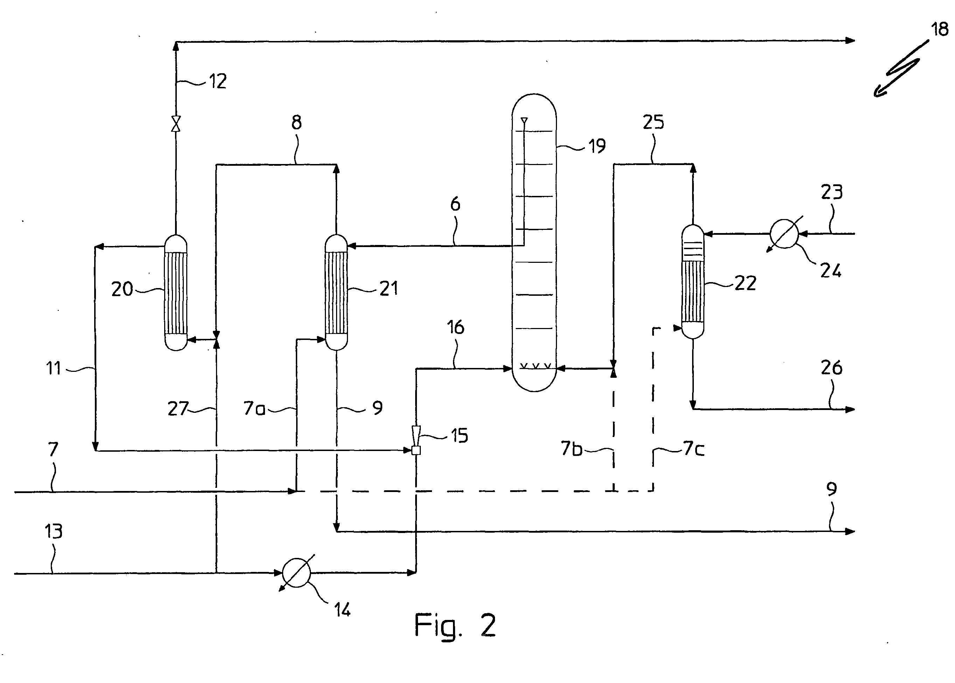

In figure 2, the details of plant 1 equivalent as for structure and operation to those illustrated in figure 1, will be indicated with the same reference numerals.

With reference to figure 2, a plant for urea production according to the present invention is indicated with reference numeral 18.

Plant 18, and more specifically the high pressure synthesis section comprises a reactor or reaction space 19 for urea synthesis, a vertical condenser 20, a first stripping unit 21 with carbon dioxide for removing most of the unreacted carbamate and free ammonia of the reaction mixture coming from the reactor 19 through the flow line 6, and a second stripping unit 22.

Moreover, plant 18 comprises a recovery section for the urea produced, which is not represented in figure 2.

The condenser 20, the first stripping unit 21 and the second stripping unit 22 generally operates at the same pressure conditions as the reactor 19.

The isobaric process pressure in the synthesis section of figure 2 is usually comprised between 140 and 160 bar.

The reactor 19 operates usually at a temperature comprised between 180°C and 200°C, with a molar ratio NH₃/CO₂ comprised between 2.5 and 4, and a molar ratio H₂O/CO₂ lower than 0.5, preferably from 0.1 to 0.2.

The first and second stripping units 21 and 22 usually operate at a temperature comprised between 160 and 210°C and the condenser 20 usually operates at a temperature comprised between 150 and 210°C.

According to the present invention, a flow of the carbamate solution coming from the urea recovery section is fed to the top of the second stripping unit 22 through the flow line 23. According to the present embodiment, the liquid flow 23 is preferably preheated in an heat exchanger 24, before entering the second stripping unit 22, by means of part of the heat removed from the carbamate condenser 20.

In the second stripping unit 22, main part of carbamate is decomposed and together with free ammonia is removed from the carbamate solution as ammonia and carbon dioxide vapors with very low content of water. The vapor flow so obtained at the outlet of the second stripping unit 22, indicated by the flow line 25, is sent to the reactor 19.

Moreover, at the outlet of the second stripping unit 22, the flow of the aqueous solution containing a residual content of carbamate and free ammonia, indicated by the flow line 26, is sent back to the urea recovery section for further processing.

The carbon dioxide feed, represented by the flow line 7, is sent, as major portion and through the flow line 7a, to the first stripping unit 21 where most of the unreacted carbamate and free ammonia coming from the reactor 19, through the flow line 6, are removed from the reaction mixture as ammonia and carbon dioxide vapors.

The gas flow so obtained, at the outlet of the first stripping unit 21, is fed, through the flow line 8, to the carbamate condenser 20.

In the condenser 20, the above gas flow is substantially totally condensed obtaining, at the outlet of the condenser 20, a carbamate solution represented by the flow line 11.

Instead, the liquid flow comprising urea and residual carbamate and free ammonia in aqueous solution leaving the first stripping unit 21, is sent, through the flow line 9, to the urea recovery section not represented in figure 2.

The remaining minor portion of carbon dioxide feed can be sent to the reactor 19, through the flow line 7b, and/or to the second stripping unit 22 through the flow line 7c.

The present invention also contemplates the possibility of sending at least part of the vapor flow obtained at the outlet of the second stripping unit 22 (flow line 25) to the first stripping unit 21 although such possibility is not shown in figure 2.

In the latter case, said vapor phase can be advantageously employed as stripping agent, either in combination with the portion of the carbon dioxide feed represented by the flow line 7a or as a separated flow, for the stripping treatment of the reaction mixture coming from the reactor 19 through the flow line 6.

The ammonia feed, represented by the flow line 13, is preheated in the heat exchanger 14 and partially sent to the ejector 15 where, as driving fluid, it provides for the recycle to the reactor 19, through flow line 16, of the carbamate solution coming from carbamate condenser 20 through the flow line 11. Appropriate means such as ducts are used for sending the ammonia feed and the carbamate solution from the ejector 15 to the reactor 19.

Preferably, the heat exchanger 14 uses part of the heat removed in the carbamate condenser 20.

The remaining portion of the ammonia feed is instead sent to the carbamate condenser 20 through the flow line 27 to promote the condensation of the ammonia and carbon dioxide vapors coming from the first stripping unit 21.

Most of the inert gases contained in the carbon dioxide feed, fed to the loop through the flow line 7, are removed from carbamate condenser head through the flow line 12.

According to a preferred embodiment a major portion (at least 90%) of carbon dioxide feed is sent to the first stripping unit 21 and the remaining minor portion is sent to the reactor 19.

In this case, the second stripping unit 22 operates by means of heat alone and the flow line 7c is missing.

Alternatively, all carbon dioxide feed can be sent to the first stripping unit 21 so that also the flow line 7b is missing.

According to another embodiment of the present invention, the above minor portion of carbon dioxide is sent to the reactor 19 and to the second stripping unit 22, which therefore operates with also carbon dioxide.

Alternatively, all carbon dioxide feed of the above minor portion can be sent to the second stripping unit 22 so that the flow line 7b is missing.

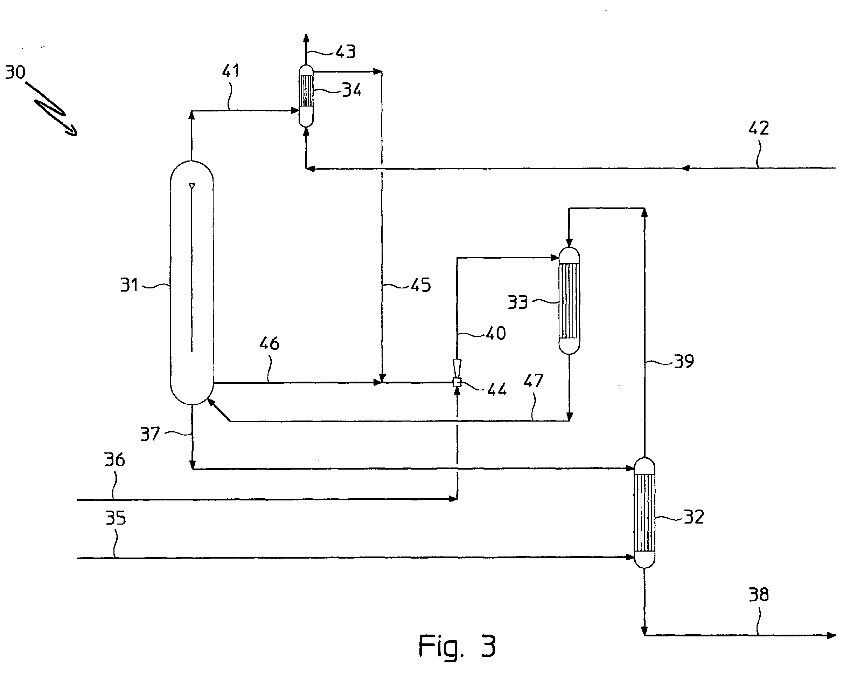

With reference to figure 3, another plant for urea production according to the stripping process with also carbon dioxide as a stripping agent and featuring the recycle of the reactants to the reaction space, is indicated with reference numeral 30.

Plant 30, and more specifically, the high pressure synthesis section, comprises a reactor 31 (or reaction space) for urea synthesis, a stripping unit 32 with carbon dioxide, a condensation section comprising a vertical condensation unit 33 of the film type, and a washing unit 34 of the passivating agents and other possible substances inert to the reaction.

With the term "condensation unit of the film type", it is intended to mean a known apparatus wherein the condensation of the gaseous phase occurs in a liquid film, flowing downwards inside a plurality of tubes in co-current with the gaseous phase. The liquid film flows within the tubes in contact with their inner wall whereas the gaseous phase flows in the free space inside the tubes.

Moreover, plant 30 comprises a recovery section for the urea produced, not represented in figure 3.

The synthesis section of plant 30 is disposed according to a so-called "vertical layout", where the reactor 31, the condensation unit 33 and the washing unit 34 are located at a higher level above the top of the stripping unit 32, so as to assure a natural circulation of the fluids in the synthesis section.

With flow lines 35 and 36 are indicated means, such as ducts, for feeding a gaseous flow comprising feed carbon dioxide at a pressure of about 150 bar to the stripping unit 32, and for feeding a flow comprising feed ammonia to the condensation unit 33, respectively.

The feed carbon dioxide sent to the stripping unit 32 through flow line 35 is employed as stripping agent of a reaction mixture comprising urea, carbamate and free ammonia in aqueous solution leaving the reactor 31 and fed to the stripping unit 32 through flow line 37.

The stripping unit 32 is of the film type with an external heating with steam.

The reaction mixture flowing downwards in the unit 32 in countercurrent with the gaseous flow comprising carbon dioxide is subjected to a treatment of partial decomposition of carbamate and partial separation of the free ammonia, obtaining a flow comprising ammonia and carbon dioxide in vapor phase and a flow comprising urea and residual carbamate in aqueous solution.

The flow comprising urea and residual carbamate in aqueous solution is extracted from the bottom of the stripping unit 32 and sent to the urea recovery section (not represented) through the flow line 38.

The gaseous flow obtained in the stripping unit 32 and comprising also small quantities of water in addition to ammonia and carbon dioxide, flows out from an upper end of such unit 32 and is fed to an upper end of the condensation unit 33 through flow line 39.

The condensation unit 33 is of the vertical film type for subjecting to partial condensation the flow comprising ammonia and carbon dioxide in vapor phase coming from the stripping unit 32 through flow line 39.

Further on, the flow comprising feed ammonia is fed to the upper end of the condensation unit 33, through ejector 44 and flow line 40, together with a recycled flow comprising ammonia and carbamate in aqueous solution coming from the washing unit 34.

Particularly, the above-mentioned recycled flow comprises carbamate in aqueous solution coming from the urea recovery section together with the carbamate obtained from the condensation, in the washing unit 34, of the ammonia and carbon dioxide in vapor phase coming from the reactor 31 through the flow line 41. Such a condensation is operated by means of a washing flow comprising the carbamate in aqueous solution coming from the urea recovery section and fed to the unit 34 through the flow line 42.

In the washing unit 34, the passivating agents and the inert substances, generally introduced in such a section through the carbon dioxide feed, are extracted from the high pressure synthesis section through the flow line 43.

The recycled flow comprising ammonia and carbamate in aqueous solution is fed into the flow comprising feed ammonia through flow line 45 and the resulting flow is fed to the condensation unit 33, as mentioned above, through ejector 44 and flow line 40. A flow line 46 is also represented in figure 3 connecting the reactor 31 to the flow line 45, for feeding carbamate from the reactor to the condensation unit

In the condensing unit 33, a partial condensation of the vapor phase comprising ammonia and carbon dioxide takes place, so obtaining a liquid phase comprising carbamate in aqueous solution as well as a gaseous phase comprising ammonia and carbon dioxide which are sent from the bottom of the condensation unit 33 to the reactor 31 through the flow line 47.

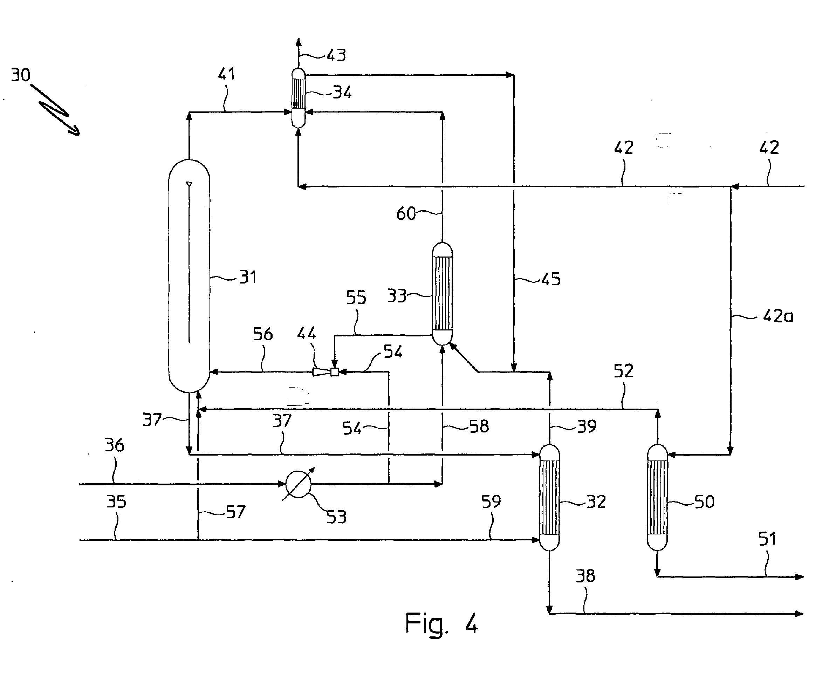

With reference to figure 4, the plant for urea production of figure 3 is advantageously represented suitably modified according to the method of modernization of the present invention.

In figure 4, the details of the plant 30 equivalent as for structure and operation to those illustrated in figure 3, will be indicated with the same reference numerals and will not be described again.

Thanks to the present method of modernization, the condensation unit 33 is advantageously modified in its internal so as to permit a substantially total condensation of the gaseous flow comprising ammonia and carbon dioxide in vapor phase coming from the stripping unit 32.

To this purpose, the existing vertical condensation unit 33 of the film type is advantageously transformed in a conventional manner in a vertical condensation unit of the "submerged type". In EP-A-1 036 787, which is incorporated herewith by reference, an example of the above transformation of the condenser is described.

This change remarkably improves the efficiency of such a unit and thus its capacity.

According to the method of modernization of the present invention, the plant 30 is also provided with a second stripping unit 50 which is fed, through a flow line 42a, with at least part of a liquid flow comprising carbamate in aqueous solution coming from the urea recovery section.

Such a liquid flow is subjected to a treatment of partial decomposition of the carbamate in the second stripping unit 50, obtaining a gaseous flow comprising ammonia and carbon dioxide in vapor phase with little quantity of water and a liquid flow comprising residual carbamate and free ammonia.

The liquid flow comprising residual carbamate and free ammonia is sent back to the urea recovery section through the flow line 51.

With regard to the gaseous flow comprising ammonia and carbon dioxide in vapor phase leaving the second stripping unit 50, the method of modernization according to the invention provides that such a gaseous flow is sent, at least in part, into the reactor 31 by suitable means, such as ducts, indicated in figure 4 with the flow line 52.

Further on, the method of modernization of the present invention provides that the ammonia feed is pre-heated by means of an heat exchanger 53 and split into a first portion and a second portion which are sent to the reactor 31 and the condensation unit 33 respectively.

Particularly, the second portion of the ammonia feed is directly fed to the condensation unit 33 through the flow line 58.

Instead, the first portion of the ammonia feed is sent, through the flow line 54, to the ejector 44 which sucks up the liquid flow comprising carbamate in aqueous solution coming from the condensation unit 33 through the flow line 55. The second portion of the ammonia feed enters the ejector 44 as driving fluid and is sent together with the above liquid flow to the reactor 31 through the flow line 56.

Further on, in the embodiment shown in figure 4, the method of modernization of the present invention provides that the carbon dioxide feed is split into two portions, which are sent respectively to the reactor 31, through the flow line 57, and to the stripping unit 32 through the flow line 59. In this embodiment, the stripping unit 50 is a thermal stripping unit.

Alternatively, all carbon dioxide feed can be sent to the first stripping unit 32 so that flow line 57 is missing.

According to another embodiment the second stripping unit 50 is a stripping unit with also carbon dioxide.

In this embodiment, the method of modernization according to the invention can provide suitable means, such as ducts, for feeding a major portion of carbon dioxide to the stripping unit 32, and suitable means, such as ducts, for feeding a remaining minor portion of carbon dioxide to the second stripping unit 50 and/or to the reactor 31 for urea synthesis.

Finally, plant 30 modernized according to the invention, also comprises suitable means, such as ducts, indicated by the flow line 60, for feeding a gaseous phase coming from the condensation unit 33 to the washing unit 34.

The above gaseous phase comprises inert and passivating agents for the urea synthesis reactor together with possible uncondensed traces of ammonia and carbon dioxide in vapor phase.

In the next examples, there are shown, by way of merely indicative and not limiting example, the conversion yields obtainable by a plant implementing the process according to the invention.

### EXAMPLE 1

In this example, the conversion yield of a reactor operating in a brand new plant implementing the process according to the present invention as described in figure 2 has been simulated.

The plant is based on the carbon dioxide stripping process, wherein 90% of the carbon dioxide to be fed to the reactor 19 is first fed, as stripping agent, in the first stripping unit 21 and the remaining 10% of the carbon dioxide is directly fed to the reactor 19. In this case, flow line 7c is missing and the second stripping unit 22 is a thermal stripping unit.

Moreover, 75% of the ammonia feed is fed to the reactor 19 through the ejector 15 whereas the remaining 25% of the ammonia feed is fed in the condenser 20 to promote the condensation of the carbamate.

Also, 75% of the carbamate solution coming from the urea recovery section is fed in the second stripping unit 22 and, according to a preferred embodiment of the invention, 100% of the vapors leaving said second stripping unit 22 are directly fed in the reaction space 19 for securing the thermal balance.

The operation conditions of the urea synthesis reactor 19 are the followings:
- molar ratio NH₃/CO₂ at input: 3.2,
- molar ratio H₂O/CO₂ at input: 0.2,
- pressure: about 150 bar,
- temperature: 188°C,
- capacity: 2000 MTD urea.

The percentage of carbon dioxide in the vapors leaving the second stripping unit 22 is 52%. The total amount of the carbon dioxide fed to the reactor 19 both directly and coming from the second stripping unit 22 is 35% of the carbon dioxide feed.

The conversion yield of the carbon dioxide into urea obtained by the present reactor is very high: 70%. Moreover, the content of water in the carbamate solution recycled to the reactor is particularly low.

In absence of the second stripping unit 22 and operating the plant under substantially the same conditions reported above in connection with the reactor 19 and the ammonia feed, about 35% of the carbon dioxide feed is to be sent to the reactor 19 to assure the thermal balance.

In this way, since not more than 65% of the carbon dioxide feed is sent to the first (only) stripping unit 21, a lower efficiency of the stripping treatment is achieved if compared to the process according to the invention and consequently lower conversion yield, higher duty in the urea recovery section and higher steam consumption.

### EXAMPLE 2

A pre-existing plant operating according to the prior art process described with reference to figure 3 is modernized in order to operate according to the process described with reference to figure 4.

The operating conditions of the urea synthesis reactor before the plant modernization are the followings:
- molar ratio NH₃/CO₂ at input: 3.0,
- molar ratio H₂O/CO₂ at input: 0.5,
- pressure: about 150 bar,
- temperature: 185°C,
- capacity: 1500 MTD urea,
- conversion yield in the reactor: 60%

After modernizing the pre-existing plant by providing a second stripping unit 50 fed with 75% of the carbamate solution coming from the urea recovery section, by feeding 90% of the carbon dioxide feed to the stripping unit 32 through the flow line 59 and the remaining 10% of the carbon dioxide feed to the reactor 31 through the flow line 57, and by feeding the vapor leaving said second stripping unit 50 directly to the reactor 31 through the flow line 52, as described with reference to figure 4, the new operating conditions of the reactor are the followings:
- molar ratio NH₃/CO₂ at input: 3.2,
- molar ratio H₂O/CO₂ at input: 0.2,
- pressure: about 150 bar,
- temperature: 188°C,
- capacity: 2250 MTD urea
- conversion yield in the reactor: 67%

The percentage of carbon dioxide in the vapors leaving the second stripping unit 50 is 50%. The total amount of the carbon dioxide fed to the reactor 19 both directly and coming from the second stripping unit 50 is 35% of the carbon dioxide feed.

Thanks to the present invention, it is possible to increase the conversion yield of 7 points percentage and to increase the capacity of 750 MTD urea, i.e. 50% more than the original capacity.

Moreover, in this case, only minor modifications to the pre-existing plant and low investment costs are required to obtain a relevant increase in the capacity and the conversion yield.

In the previous description and in the subsequent claims, by the expressions: "means for feeding", "means for recycling" and "means for sending", it is intended to indicate connecting ducts, pumps, valves ejectors and other conventional devices for bringing fluids from one point to another of the plant.

## Claims

1. A process for the production of urea of the type comprising the steps of:
- performing a reaction between ammonia and carbon dioxide in a reaction space to obtain a reaction mixture comprising urea, carbamate and free ammonia in aqueous solution,
- subjecting said mixture to a stripping treatment with the use also of carbon dioxide feed as a stripping agent to obtain a first flow comprising ammonia and carbon dioxide in vapor phase and a flow comprising urea and residual carbamate in aqueous solution,
- subjecting said first flow comprising ammonia and carbon dioxide in vapor phase to a substantially total condensation to obtain a first portion of carbamate in aqueous solution,
- recycling said first portion of carbamate in aqueous solution to said reaction space,
- feeding said flow comprising urea and residual carbamate in aqueous solution to a urea recovery section,
- separating in said recovery section said residual carbamate from the urea to obtain a second portion of carbamate in aqueous solution.
**characterized in that** it comprises the additional steps of:
- subjecting at least part of said second portion of carbamate in aqueous solution obtained in said urea recovery section to a treatment of partial decomposition to obtain a second flow comprising ammonia and carbon dioxide in vapor phase and a flow comprising residual carbamate in aqueous solution,
- recycling at least part of said second flow comprising ammonia and carbon dioxide in vapor phase to said reaction space and/or using at least part of said second flow comprising ammonia and carbon dioxide in vapor phase for the stripping treatment of the reaction mixture comprising urea, carbamate and free ammonia in aqueous solution.

2. A process according to claim 1, **characterized in that** the treatment of partial decomposition of the said at least part of the second portion of carbamate in aqueous solution is carried out at a pressure substantially corresponding to the pressure in the reaction space.

3. A process according to claim 1 or claim 2, **characterized in that** it further comprises the step of feeding the flow comprising residual carbamate in aqueous solution resulting from the treatment of partial decomposition of at least part of the second portion of carbamate to said urea recovery section.

4. A plant (18) for urea production comprising:
- a urea synthesis reactor (19) for performing a reaction between ammonia and carbon dioxide obtaining a reaction mixture comprising urea, carbamate and free ammonia in aqueous solution,
- a first thermal stripping unit (21) with carbon dioxide as a stripping agent for subjecting said reaction mixture to a treatment of partial decomposition of the carbamate and partial separation of the free ammonia in aqueous solution present in said mixture, obtaining a first flow comprising ammonia and carbon dioxide in vapor phase and a flow comprising urea and residual carbamate in aqueous solution,
- means (20) for substantially totally condensing said first flow comprising ammonia and carbon dioxide in vapor phase obtaining a first portion of carbamate in aqueous solution,
- means (11,15,16) for recycling said first portion of carbamate in aqueous solution to said reactor (19),
- a urea recovery section for separating urea from said flow comprising urea and residual carbamate in aqueous solution leaving said first stripping unit (21), obtaining a second portion of carbamate in aqueous solution,
which plant is **characterized in that** it comprises:
- a second stripping unit (22) for subjecting at least part of said second portion of carbamate in aqueous solution to a treatment of partial decomposition, obtaining a second flow comprising ammonia and carbon dioxide in vapor phase,
- means (25) for recycling at least part of said second flow comprising ammonia and carbon dioxide in vapor phase to said reactor (19) and/or to said first stripping unit (21).

5. A plant according to claim 4 **characterized in that** said means for totally condensing the first flow comprising ammonia and carbon dioxide in vapor phase comprises a carbamate condenser (20) of the submerged type.

6. A plant according to claim 5 **characterized in that** it further comprises:
means (13,15,16) for feeding a first portion of liquid ammonia in said reactor (19) for urea synthesis, and
means (13,27) for feeding a second portion of liquid ammonia to said carbamate condenser (20).

7. A plant according to claim 6 **characterized in that** it further comprises:
- means (24) for preheating said at least part of the second portion of carbamate solution to be sent to the second stripping unit (22).

8. A plant according to claim 6 **characterized in that** it further comprises:
- means (14) for preheating said first portion of liquid ammonia to be sent to the urea synthesis reactor (19) and/or said second portion of liquid ammonia to be sent to the carbamate condenser (20).

9. A plant according to claim 6 **characterized in that** said means (13,15,16) for feeding the first portion of liquid ammonia to said reactor (19) and said means (11,15,16) for recycling a first portion of carbamate in aqueous solution to said reactor (19) comprise:
an ejector (15),
means (11) for feeding said first portion of carbamate in aqueous solution to the ejector (15),
means (13) for feeding said first portion of liquid ammonia to the ejector (15), and
means (16) for sending said first portion of carbamate in aqueous solution together with said first portion of liquid ammonia from the ejector (15) to the reactor (19) for urea synthesis.

10. A plant according to anyone of preceding claims from 4 to 9, **characterized in that** said second stripping unit (22) is a thermal stripping unit.

11. A plant according to claim 10 **characterized in that** it further comprises means (7,7a) for feeding all carbon dioxide feed to said first stripping unit (21).

12. A plant according to claim 10 **characterized in that** it further comprises:
- means (7, 7a) for feeding a major portion of carbon dioxide feed to said first stripping unit (21), and
- means (7,7b) for feeding a remaining minor portion of carbon dioxide feed to said reactor (19) for urea synthesis.

13. A plant according to anyone of preceding claims from 4 to 9, **characterized in that** said second stripping unit (22) is a thermal stripping unit with also carbon dioxide as a stripping agent.

14. A plant according to claim 13 **characterized in that** it further comprises:
- means (7,7a) for feeding a major portion of carbon dioxide feed to said first stripping unit (21), and
- means (7,7b,7c) for feeding a remaining minor portion of carbon dioxide feed to said second stripping unit (22) and/or to said reactor . (19) for urea synthesis.

15. A process according to claim 1, **characterized in that** said reaction between ammonia and carbon dioxide is carried out in a reactor (19); said stripping treatment is carried out in a first thermal stripping unit (21) with also carbon dioxide as a stripping agent, subjecting said mixture to a treatment of partial decomposition of carbamate and partial separation of free ammonia; said substantially total condensation is carried out in a carbamate condenser (20); said recycling of said first portion of carbamate to said reactor (19) is carried out by means of an ejector (15) which use liquid ammonia as driving fluid; said treatment of partial decomposition of said at least part of said second portion of carbamate in aqueous solution is carried out in a second stripping unit (22); said at least part of said second flow comprising ammonia and carbon dioxide in vapor phase is recycled to the reactor (19) and/or to the first stripping unit (21).

16. A process according to claim 15 **characterized in that** the treatment of partial decomposition of said at least part of the second portion of carbamate in aqueous solution is carried out in the second stripping unit (22) at a pressure substantially corresponding to the pressure in the reactor (19).

17. A process according to claim 16 **characterized in that** it further comprises the step of:
- feeding the flow comprising residual carbamate in aqueous solution resulting from the treatment of partial decomposition of the second portion of carbamate to said urea recovery section.

18. A process according to claim 17 **characterized in that** it further comprises the steps of:
- feeding a first portion of liquid ammonia in said reactor (19) for urea synthesis, and
- feeding a second portion of liquid ammonia to said carbamate condenser (20).

19. A process according to claim 18 **characterized in that** said second stripping unit (22) is a thermal stripping unit and **in that** it further comprises the step of feeding all carbon dioxide to said first stripping unit (21).

20. A process according to claim 18 **characterized in that** said second stripping unit (22) is a thermal stripping unit and **in that** it further comprises the steps of:
- feeding a major portion of carbon dioxide feed to said first stripping unit (21), and
- feeding a remaining minor portion of carbon dioxide to said reactor (19) for urea synthesis.

21. A process according to claim 18 **characterized in that** said second stripping unit (22) is a thermal stripping unit with also carbon dioxide as a stripping agent and **in that** it further comprises the steps of:
- feeding a major portion of carbon dioxide to said first stripping unit (21),
- feeding a remaining minor portion of carbon dioxide to said second stripping unit (22) and/or to said reactor (19) for urea synthesis.

22. A process according to claim 20 or claim 21 **characterized in that** said major portion of carbon dioxide feed is at least 90% of the whole carbon dioxide feed.

23. A method for modernizing a urea production plant of the type comprising:
- a reactor (31) for urea synthesis;
- a first thermal stripping unit (32) with also carbon dioxide as a stripping agent for subjecting a reaction mixture comprising urea, carbamate and free ammonia in aqueous solution leaving the reactor (31) to a treatment of partial decomposition of carbamate and partial separation of carbamate and partial separation of free ammonia, thus obtaining a first flow comprising ammonia and carbon dioxide in vapor phase and a flow comprising urea and residual carbamate in aqueous solution, respectively,
- a urea recovery section for separating urea from said flow comprising urea and residual carbamate in aqueous solution leaving the first stripping unit (32), obtaining a second portion of carbamate in aqueous solution,
- at least one condensation unit (33) for subjecting to partial condensation the first flow comprising ammonia and carbon dioxide in vapor phase leaving the first stripping unit (32), thus obtaining a liquid flow comprising carbamate in aqueous solution and a gaseous flow comprising ammonia and carbon dioxide in vapor phase,
- means for respectively feeding the flow comprising carbamate in aqueous solution and the gaseous flow comprising ammonia and carbon dioxide in vapor phase to the reactor (31) for urea synthesis,
said modernization method being **characterized in that** it comprises the steps of:
- transforming said at least one condensation unit (33) in a total condensation unit for subjecting to substantially total condensation said first flow comprising ammonia and carbon dioxide in vapor phase, obtaining a liquid flow comprising urea and carbamate in aqueous solution,
- providing a second stripping unit (50) for subjecting at least part of said second portion of carbamate in aqueous solution to a treatment of partial decomposition, obtaining a second flow comprising ammonia and carbon dioxide in vapor phase,
- providing means for recycling at least part of the second flow comprising ammonia and carbon dioxide in vapor phase to the reactor (31) and/or to the first stripping unit (32).

24. A method according to claim 23 in which said condensation unit (33) for subjecting to partial condensation the first flow comprising ammonia and carbon dioxide in vapor phase leaving the first stripping unit (32) is of the film type and it is transformed in a condensation unit of the submerged type.

25. A method according to claim 24 **characterized in that** it further comprises:
- means (54,44,56) for feeding a first portion of liquid ammonia in said reactor (31) for urea synthesis, and
- means (58) for feeding a second portion of liquid ammonia to said condensation unit (33).

26. A method according to claim 25 **characterized in that** said second stripping unit (50) is a thermal stripping unit.

27. A method according to claim 26 **characterized in that** it further comprises means for feeding all carbon dioxide feed to said first stripping unit (32).

28. A method according to claim 26 **characterized in that** it further comprises:
- means (59) for feeding a major portion of carbon dioxide feed to said first stripping unit (32), and
- means (57) for feeding a remaining minor portion of carbon dioxide feed to said reactor (31) for urea synthesis.

29. A method according to claim 25 **characterized in that** said second stripping unit (50) is a thermal stripping unit with also carbon dioxide as a stripping agent.

30. A method according to claim 29 **characterized in that** it further comprises:
- means (59) for feeding a major portion of carbon dioxide feed to said first stripping unit (32), and
- means for feeding a remaining minor portion of carbon dioxide feed to said second stripping unit (50) and/or to said reactor (31) for urea synthesis.

## Patentansprüche

1. Prozess zur Herstellung von Harnstoff, von der Art, die die Schritte umfasst:
- Ausführen einer Reaktion zwischen Ammoniak und Kohlendioxid in einem Reaktionsraum, um ein Reaktionsgemisch zu erhalten, das Harnstoff, Carbamat und freies Ammoniak in wässriger Lösung enthält,
- Unterziehen des Gemischs einer Abziehbehandlung, wobei auch die Kohlendioxideinspeisung als Abziehmittel verwendet wird, um einen ersten Strom zu erhalten, der Ammoniak und Kohlendioxid in der Dampfphase enthält, und einen Strom, der Harnstoff und Restcarbamat in wässriger Lösung enthält,
- Unterziehen des ersten Stroms, der Ammoniak und Kohlendioxid in der Dampfphase enthält, einer im Wesentlichen vollständigen Kondensation, um einen ersten Anteil des Carbamats in wässriger Lösung zu erhalten,
- Wiedereinleiten des ersten Anteils des Carbamats in wässriger Lösung in den Reaktionsraum,
- Einleiten des Stroms, der Harnstoff und Restcarbamat in wässriger Lösung enthält, in einen Harnstoffrückgewinnungsabschnitt,
- im Rückgewinnungsabschnitt stattfindendes Abtrennen des Restcarbamats vom Harnstoff, um einen zweiten Anteil des Carbamats in wässriger Lösung zu erhalten,
**dadurch gekennzeichnet, dass** es die zusätzlichen Schritte umfasst:
- Unterziehen wenigstens eines Teils des zweiten Anteils des Carbamats in wässriger Lösung, der im Harnstoffrückgewinnungsabschnitt gewonnen wurde, einer Behandlung einer teilweisen Zersetzung, um einen zweiten Strom, der Ammoniak und Kohlendioxid in der Dampfphase enthält, und einen Strom zu erhalten, der Restcarbamat in wässriger Lösung enthält,
- Wiedereinleiten zumindest eines Teils des zweiten Stroms, der Ammoniak und Kohlendioxid in der Dampfphase enthält, in den Reaktionsraum und/oder Verwenden zumindest eines Teils des zweiten Stroms, der Ammoniak und Kohlendioxid in der Dampfphase enthält, für die Abziehbehandlung des Reaktionsgemischs, das Harnstoff, Carbamat und freies Ammoniak in wässriger Lösung enthält.

2. Prozess nach Anspruch 1, **dadurch gekennzeichnet, dass** die Behandlung der teilweisen Zersetzung von zumindest des Teils des zweiten Anteils des Carbamats in wässriger Lösung bei einem Druck ausgeführt wird, der im Wesentlichen dem Druck im Reaktionsraum entspricht.

3. Prozess nach Anspruch 1 oder Anspruch 2, **dadurch gekennzeichnet, dass** es darüber hinaus den Schritt umfasst, den Strom, der Restcarbamat in wässriger Lösung enthält, der sich aus der Behandlung der teilweisen Zersetzung von zumindest einem Teil des zweiten Anteils des Carbamats ergibt, in den Harnstoffrückgewinnungsabschnitt einzuleiten.

4. Anlage (18) zur Harnstoffherstellung, umfassend:
- einen Harnstoffsynthesereaktor (19) zum Ausführen einer Reaktion zwischen Ammoniak und Kohlendioxid, wobei ein Reaktionsgemisch erhalten wird, das Harnstoff, Carbamat und freies Ammoniak in wässriger Lösung enthält,
- eine erste thermische Abzieheinheit (21) mit Kohlendioxid als Abziehmittel, um das Reaktionsgemisch einer Behandlung einer teilweisen Zersetzung des Carbamats und einer teilweisen Abtrennung des in dem Gemisch vorhandenen freien Ammoniaks in wässriger Lösung zu unterziehen, wobei ein erster Strom, der Ammoniak und Kohlendioxid in der Dampfphase enthält, und ein Strom erhalten wird, der Harnstoff und Restcarbamat in wässriger Lösung enthält,
- Einrichtungen (20), um den ersten Strom, der Ammoniak und Kohlendioxid in der Dampfphase enthält, im Wesentlichen vollständig zu kondensieren, wobei ein erster Anteil des Carbamats in wässriger Lösung erhalten wird,
- Einrichtungen (11, 15, 16) zum Wiedereinleiten des ersten Anteils des Carbamats in wässriger Lösung in den Reaktor (19),
- einen Harnstoffrückgewinnungsabschnitt zum Abtrennen von Harnstoff aus dem Strom, der Harnstoff und Restcarbamat in wässriger Lösung enthält und die erste Abzieheinheit (21) verlässt, wobei ein zweiter Anteil des Carbamats in wässriger Lösung erhalten wird,
welche Anlage **dadurch gekennzeichnet ist, dass** sie umfasst:
- eine zweite Abzieheinheit (22) zum Unterziehen wenigstens eines Teils des zweiten Anteils des Carbamats in wässriger Lösung einer Behandlung einer teilweisen Zersetzung, wobei ein zweiter Strom erhalten wird, der Ammoniak und Kohlendioxid in der Dampfphase enthält,
- Einrichtungen (25) zum Wiedereinleiten zumindest eines Teils des zweiten Stroms, der Ammoniak und Kohlendioxid in der Dampfphase enthält, in den Reaktionsraum (19) und/oder die erste Abzieheinheit (21).

5. Anlage nach Anspruch 4, **dadurch gekennzeichnet, dass** die Einrichtungen zur vollständigen Kondensation des ersten Stroms, der Ammoniak und Kohlendioxid in der Dampfphase enthält, einen Carbamatkondensator (20) in Eintauchbauart umfasst.

6. Anlage nach Anspruch 5, **dadurch gekennzeichnet, dass** sie darüber hinaus umfasst:
- Einrichtungen (13, 15, 16) zum Einleiten eines ersten Anteils flüssigen Ammoniaks in den Reaktor (19) zur Harnstoffsynthese, und
- Einrichtungen (13, 27) zum Einleiten eines zweiten Anteils flüssigen Ammoniaks in den Carbamatkondensator (20).

7. Anlage nach Anspruch 6, **dadurch gekennzeichnet, dass** sie darüber hinaus umfasst:
- Einrichtungen (24) zum Vorwärmen zumindest eines Teils des zweiten Anteils der Carbamatlösung, die zur zweiten Abzieheinheit (22) zu befördern ist.

8. Anlage nach Anspruch 6, **dadurch gekennzeichnet, dass** sie darüber hinaus umfasst:
- Einrichtungen (14) zum Vorwärmen des ersten Anteils des flüssigen Ammoniaks, der in den Harnstoffsynthesereaktor (19) zu befördern ist, und/oder des zweiten Anteils des flüssigen Ammoniaks, der in den Carbamatkondensator (20) zu befördern ist.

9. Anlage nach Anspruch 6, **dadurch gekennzeichnet, dass** die Einrichtungen (13, 15, 16) zum Einleiten des ersten Anteils des flüssigen Ammoniaks in den Reaktor (19) und die Einrichtungen (11, 15, 16) zum Wiedereinleiten eines ersten Anteils des Carbamats in wässriger Lösung in den Reaktor (19) umfassen:
eine Ausstoßvorrichtung (15),
Einrichtungen (11) zum Einleiten des ersten Anteils des Carbamats in wässriger Lösung in die Ausstoßvorrichtung (15),
Einrichtungen (13) zum Einleiten des ersten Anteils des flüssigen Ammoniaks in die Ausstoßvorrichtung (15), und
- Einrichtungen (16) zum Befördern des ersten Anteils des Carbamats in wässriger Lösung zusammen mit dem ersten Anteil des flüssigen Ammoniaks von der Ausstoßvorrichtung (15) zum Reaktor (19) zur Harnstoffsynthese.

10. Anlage nach einem der vorhergehenden Ansprüche 4 bis 9, **dadurch gekennzeichnet, dass** die zweite Abzieheinheit (22) eine thermische Abzieheinheit ist.

11. Anlage nach Anspruch 10, **dadurch gekennzeichnet, dass** sie darüber hinaus Einrichtungen (7, 7a) zum Einleiten der gesamten Kohlendioxideinspeisung in die erste Abzieheinheit (21) umfasst.

12. Anlage nach Anspruch 10, **dadurch gekennzeichnet, dass** sie darüber hinaus umfasst:
- Einrichtungen (7, 7a) zum Einleiten eines Hauptanteils der Kohlendioxideinspeisung in die erste Abzieheinheit (21), und
- Einrichtungen (7, 7b) zum Einleiten eines verbleibenden kleineren Anteils der Kohlendioxideinspeisung in den Reaktor (19) zur Harnstoffsynthese.

13. Anlage nach einem der vorhergehenden Ansprüche 4 bis 9, **dadurch gekennzeichnet, dass** die zweite Abzieheinheit (22) eine thermische Abzieheinheit ist, die auch mit Kohlendioxid als Abziehmittel arbeitet.

14. Anlage nach Anspruch 13, **dadurch gekennzeichnet, dass** sie darüber hinaus umfasst:
- Einrichtungen (7, 7a) zum Einleiten eines Hauptanteils der Kohlendioxideinspeisung in die erste Abzieheinheit (21), und
- Einrichtungen (7, 7b, 7c) zum Einleiten eines verbleibenden kleineren Anteils der Kohlendioxideinspeisung in die zweite Abzieheinheit (22) und/oder den Reaktor (19) zur Harnstoffsynthese.

15. Prozess nach Anspruch 1, **dadurch gekennzeichnet, dass** die Reaktion zwischen Ammoniak und Kohlendioxid in einem Reaktor (19) ausgeführt wird; die Abziehbehandlung in einer ersten Abzieheinheit (21) auch mit Kohlendioxid als Abziehmittel durchgeführt wird, wobei das Gemisch einer Behandlung einer teilweisen Zersetzung des Carbamats und einer teilweisen Abtrennung des freien Ammoniaks unterworfen wird; die im Wesentlichen vollständige Kondensation in einem Carbamatkondensator (20) durchgeführt wird; das Wiedereinleiten des ersten Anteils des Carbamats in den Reaktor (19) mittels einer Ausstoßvorrichtung (15) ausgeführt wird, die flüssiges Ammoniak als Antriebsfluid verwendet; die Behandlung der teilweisen Zersetzung von zumindest dem Teil des zweiten Anteils des Carbamats in wässriger Lösung in einer zweiten Abzieheinheit (22) durchgeführt wird; zumindest der Teil des zweiten Stroms, der Ammoniak und Kohlendioxid in der Dampfphase enthält, in den Reaktor (19) und/oder die erste Abzieheinheit (21) wiedereingeleitet wird.

16. Prozess nach Anspruch 15, **dadurch gekennzeichnet, dass** die Behandlung der teilweisen Zersetzung von zumindest dem Teil des zweiten Anteils des Carbamats in wässriger Lösung in der zweiten Abzieheinheit (22) bei einem Druck ausgeführt wird, der im Wesentlichen dem Druck im Reaktor (19) entspricht.

17. Prozess nach Anspruch 16, **dadurch gekennzeichnet, dass** er darüber hinaus den Schritt umfasst:
- Einleiten des Stroms, der Restcarbamat in wässriger Lösung enthält, der sich aus der Behandlung der teilweisen Zersetzung des zweiten Anteils des Carbamats ergibt, in den Harnstoffrückgewinnungsabschnitt.

18. Prozess nach Anspruch 17, **dadurch gekennzeichnet, dass** er darüber hinaus die Schritte umfasst:
- Einleiten eines ersten Anteils des flüssigen Ammoniaks in den Reaktor (19) zur Harnstoffsynthese, und
Einleiten eines zweiten Anteils des flüssigen Ammoniaks in den Carbamatkondensator (20).

19. Prozess nach Anspruch 18, **dadurch gekennzeichnet, dass** die zweite Abzieheinheit (22) eine thermische Abzieheinheit ist, und er darüber hinaus den Schritt umfasst, das gesamte Kohlendioxid in die erste Abzieheinheit (21) einzuleiten.

20. Prozess nach Anspruch 18, **dadurch gekennzeichnet, dass** die zweite Abzieheinheit (22) eine thermische Abzieheinheit ist, und er darüber hinaus die Schritte umfasst:
- Einleiten eines Hauptanteils der Kohlendioxideinspeisung in die erste Abzieheinheit (21), und
- Einleiten eines verbleibenden kleinern Anteils des Kohlendioxids in den Reaktor (19) zur Harnstoffsynthese.

21. Prozess nach Anspruch 18, **dadurch gekennzeichnet, dass** die zweite Abzieheinheit (22) eine thermische Abzieheinheit ist, die auch mit Kohlendioxid als Abziehmittel arbeitet, und dass er darüber hinaus die Schritte umfasst:
- Einleiten eines Hauptanteils des Kohlendioxids in die erste Abzieheinheit (21),
- Einleiten eines verbleibenden kleineren Anteils des Kohlendioxids in die zweite Abzieheinheit (22) und/oder den Reaktor (19) zur Harnstoffsynthese.

22. Prozess nach Anspruch 20 oder 21, **dadurch gekennzeichnet, dass** der Hauptanteil der Kohlendioxideinspeisung mindestens 90% der gesamten Kohlendioxideinspeisung beträgt.

23. Verfahren zur Modernisierung einer Harnstoffherstellungsanlage der Art, die umfasst:
- einen Reaktor (31) zur Harnstoffsynthese;
- eine erste thermische Abzieheinheit (32), die auch mit Kohlendioxid als Abziehmittel arbeitet, um ein Reaktionsgemisch, das Harnstoff, Carbamat und freies Ammoniak in wässriger Lösung enthält und den Reaktor (31) verlässt, einer Behandlung einer teilweisen Zersetzung des Carbamats und einer teilweisen Abtrennung des Carbamats und einer teilweisen Abtrennung des freien Ammoniaks zu unterziehen, womit ein erster Strom, der Ammoniak und Kohlendioxid in der Dampfphase enthält, bzw. ein Strom erhalten wird, der Harnstoff und Restcarbamat in wässriger Lösung enthält,
- einen Harnstoffrückgewinnungsabschnitt zum Abtrennen von Harnstoff aus dem Strom, der Harnstoff und Restcarbamat in wässriger Lösung enthält und die erste Abzieheinheit (32) verlässt, wobei ein zweiter Anteil des Carbamats in wässriger Lösung erhalten wird,
- zumindest eine Kondensationseinheit (33), um den ersten Strom, der Ammoniak und Kohlendioxid in der Dampfphase enthält und die erste Abzieheinheit (32) verlässt, einer teilweisen Kondensation zu unterziehen, womit ein flüssiger Strom erhalten wird, der Carbamat in wässriger Lösung enthält, und ein gasförmiger Strom erhalten wird, der Ammoniak und Kohlendioxid in der Dampfphase enthält,
- Einrichtungen zum jeweiligen Einleiten des Stroms, der Carbamat in wässriger Lösung enthält, und des gasförmigen Stroms, der Ammoniak und Kohlendioxid in der Dampfphase enthält, in den Reaktor (31) zur Harnstoffsynthese,
wobei das Modernisierungsverfahren **dadurch gekennzeichnet ist, dass** es die Schritte umfasst:
- Umwandeln der zumindest einen Kondensationseinheit (33) in eine Kondensationseinheit für vollständige Kondensation, um den ersten Strom, der Ammoniak und Kohlendioxid in der Dampfphase enthält, einer im Wesentlichen vollständigen Kondensation zu unterziehen, wobei ein flüssiger Strom erhalten wird, der Harnstoff und Carbamat in wässriger Lösung enthält,
- Vorsehen einer zweiten Abzieheinheit (50), um zumindest einen Teil des zweiten Anteils des Carbamats in wässriger Lösung einer Behandlung einer teilweisen Zersetzung zu unterziehen, wobei ein zweiter Strom erhalten wird, der Ammoniak und Kohlendioxid in der Dampfphase enthält,
- Vorsehen von Einrichtungen zum Wiedereinleiten zumindest eines Teils des zweiten Stroms, der Ammoniak und Kohlendioxid in der Dampfphase enthält, in den Reaktor (31) und/oder die erste Abzieheinheit (32).

24. Verfahren nach Anspruch 23, bei dem die Kondensationseinheit (33), die den ersten Strom, der Ammoniak und Kohlendioxid in der Dampfphase enthält und die erste Abzieheinheit (32) verlässt, einer teilweisen Kondensation unterzieht, in Dünnschichtbauart vorliegt und in eine Kondensationseinheit der Eintauchbauart umgewandelt wird.

25. Verfahren nach Anspruch 24, **dadurch gekennzeichnet, dass** es darüber hinaus umfasst:
- Einrichtungen (54, 44, 56) zum Einleiten eines ersten Anteils des flüssigen Ammoniaks in den Reaktor (31) zur Harnstoffsynthese, und
- Einrichtungen (58) zum Einleiten eines zweiten Anteils des flüssigen Ammoniaks in die Kondensationseinheit (33).

26. Verfahren nach Anspruch 25, **dadurch gekennzeichnet, dass** die zweite Abzieheinheit (50) eine thermische Abzieheinheit ist.

27. Verfahren nach Anspruch 26, **dadurch gekennzeichnet, dass** es darüber hinaus Einrichtungen zum Einleiten der gesamten Kohlendioxideinspeisung in die erste Abzieheinheit (32) umfasst.

28. Verfahren nach Anspruch 26, **dadurch gekennzeichnet, dass** es darüber hinaus umfasst:
- Einrichtungen (59) zum Einleiten eines Hauptanteils der Kohlendioxideinspeisung in die erste Abzieheinheit (32), und
- Einrichtungen (57) zum Einleiten eines verbleibenden kleineren Anteils der Kohlendioxideinspeisung in den Reaktor (31) zur Harnstoffsynthese.

29. Verfahren nach Anspruch 25, **dadurch gekennzeichnet, dass** die zweite Abzieheinheit (50) eine thermische Abzieheinheit ist, die auch mit Kohlendioxid als Abziehmittel arbeitet.

30. Verfahren nach Anspruch 29, **dadurch gekennzeichnet, dass** es darüber hinaus umfasst:
- Einrichtungen (59) zum Einleiten eines Hauptanteils der Kohlendioxideinspeisung in die erste Abzieheinheit (32), und
- Einrichtungen zum Einleiten eines verbleibenden kleineren Anteils der Kohlendioxideinspeisung in die zweite Abzieheinheit (50) und/oder den Reaktor (31) zur Harnstoffsynthese.

## Revendications

1. Procédé de production d'urée du type comprenant les étapes qui consistent à :
- mettre en oeuvre une réaction entre de l'ammoniac et du dioxyde de carbone dans un espace réactionnel afin d'obtenir un mélange réactionnel comprenant de l'urée, un carbamate et de l'ammoniac libre en solution aqueuse,
- soumettre ledit mélange à un traitement d'extraction à l'aide également d'un apport de dioxyde de carbone en tant qu'agent d'extraction afin d'obtenir un premier flux comprenant de l'ammoniac et du dioxyde de carbone en phase vapeur et un flux comprenant de l'urée et un carbamate résiduel en solution aqueuse,
- soumettre ledit premier flux comprenant de l'ammoniac et du dioxyde de carbone en phase vapeur à une condensation sensiblement totale afin d'obtenir une première portion de carbamate en solution aqueuse,
- recycler ladite première portion de carbamate en solution aqueuse dans ledit espace réactionnel,
- introduire ledit flux comprenant de l'urée et le carbamate résiduel en solution aqueuse dans une section de récupération d'urée,
- séparer dans ladite section de récupération ledit carbamate résiduel de l'urée afin d'obtenir une seconde portion de carbamate en solution aqueuse,
**caractérisé en ce qu'**il comprend les étapes supplémentaires consistant à :
- soumettre au moins une partie de ladite seconde portion de carbamate en solution aqueuse obtenue dans ladite section de récupération d'urée à un traitement de décomposition partielle afin d'obtenir un second flux comprenant de l'ammoniac et du dioxyde de carbone en phase vapeur et un flux comprenant un carbamate résiduel en solution aqueuse,
- recycler au moins une partie dudit second flux comprenant de l'ammoniac et du dioxyde de carbone en phase vapeur dans ledit espace réactionnel et/ou utiliser au moins une partie dudit second flux comprenant de l'ammoniac et du dioxyde de carbone en phase vapeur pour le traitement d'extraction du mélange réactionnel comprenant de l'urée, un carbamate et de l'ammoniac libre en solution aqueuse.

2. Procédé selon la revendication 1, **caractérisé en ce que** le traitement de décomposition partielle de ladite ou des dites parties de la seconde portion de carbamate en solution aqueuse est mis en oeuvre à une pression correspondant sensiblement à la pression existant dans l'espace réactionnel.

3. Procédé selon la revendication 1 ou la revendication 2, **caractérisé en ce qu'**il comprend en outre l'étape consistant à introduire le flux comprenant un carbamate résiduel en solution aqueuse résultant du traitement de la décomposition partielle d'au moins une partie de la seconde portion de carbamate dans ladite section de récupération d'urée.

4. Installation (18) pour la production d'urée comprenant :
- un réacteur de synthèse (19) d'urée destiné à la mise en oeuvre d'une réaction entre de l'ammoniac et du dioxyde de carbone permettant d'obtenir un mélange réactionnel comprenant de l'urée, un carbamate et de l'ammoniac libre en solution aqueuse,
- une première unité d'extraction thermique (21) comportant du dioxyde de carbone en tant qu'agent d'extraction destinée à soumettre ledit mélange réactionnel à un traitement de décomposition partielle du carbamate et de séparation partielle de l'ammoniac libre en solution aqueuse présents dans ledit mélange, permettant d'obtenir un premier flux comprenant de l'ammoniac et du dioxyde de carbone en phase vapeur et un flux comprenant de l'urée et un carbamate résiduel en solution aqueuse,
- un moyen (20) destiné à condenser sensiblement totalement ledit premier flux comprenant de l'ammoniac et du dioxyde de carbone en phase vapeur, permettant d'obtenir une première portion de carbamate en solution aqueuse,
- des moyens (11, 15, 16) destinés à recycler ladite première portion de carbamate en solution aqueuse dans ledit réacteur (19),
- une section de récupération d'urée destinée à séparer l'urée dudit flux comprenant de l'urée et du carbamate résiduel en solution aqueuse sortant de ladite première unité d'extraction (21), permettant d'obtenir une seconde portion de carbamate en solution aqueuse,
laquelle installation est **caractérisé en ce qu'**elle comprend :
- une seconde unité d'extraction (22) destinée à soumettre au moins une partie de ladite seconde portion de carbamate en solution aqueuse à un traitement de décomposition partielle, permettant d'obtenir un second flux comprenant de l'ammoniac et du dioxyde de carbone en phase vapeur,
- un moyen (25) destiné à recycler au moins une partie dudit second flux comprenant de l'ammoniac et du dioxyde de carbone en phase vapeur dans ledit réacteur (19) et/ou dans ladite première unité d'extraction (2 1).

5. Installation selon la revendication4, **caractérisée en ce que** ledit moyen destiné à condenser totalement le premier flux comprenant de l'ammoniac et du dioxyde de carbone en phase vapeur comprend un condensateur de carbamate (20) du type submergé,

6. Installation selon la revendication 5, **caractérisée en ce qu'**elle comprend en outre :
des moyens (13, 15, 16) destinés à introduire une première portion d'ammoniac liquide dans ledit réacteur (19) pour une synthèse d'urée, et
des moyens (13, 27) destinés à introduire une seconde portion d'ammoniac liquide dans ledit condensateur de carbamate (20).

7. Installation selon la revendication 6, **caractérisée en ce qu'**elle comprend en outre :
- des moyens (24) destinés à préchauffer ladite ou lesdites parties de la seconde portion de la solution de carbamate à introduire dans la seconde unité d'extraction (22).

8. Installation selon la revendication 6, **caractérisée en ce qu'**elle comprend en outre :
- des moyens (14) destinés à préchauffer ladite première portion d'ammoniac liquide devant être introduite dans le réacteur de synthèse (19) d'urée et/ou ladite seconde portion d'ammoniac liquide devant être introduite dans le condensateur de carbamate (20).

9. Installation selon la revendication 6, **caractérisée en ce que** les dits moyens (13, 15, 16) destinés à introduire la première portion de l'ammoniac liquide dans ledit réacteur (19) et lesdits moyens (11, 15, 16) destinés à recycler une première portion de carbamate en solution aqueuse dans ledit réacteur (19) comprennent :
un éjecteur (15),
des moyens (11) destinés à introduire ladite première portion de carbamate en solution aqueuse dans l'éjecteur (15),
un moyen (13) destiné à introduire ladite première portion d'ammoniac liquide dans l'éjecteur (15), et
des moyens (16) destinés à introduire ladite première portion de carbamate en solution aqueuse conjointement avec ladite première portion d'ammoniac liquide provenant de l'éjecteur (15) dans le réacteur (19) pour une synthèse d'urée.

10. Installation selon l'une quelconque des revendications précédentes 4 à 9, **caractérisée en ce que** ladite seconde unité d'extraction (22) est une unité d'extraction thermique.

11. Installation selon la revendication 10, **caractérisée en ce qu'**elle comprend en outre des moyens (7, 7a) destinés à introduire tout l'apport de dioxyde de carbone dans ladite première unité d'extraction (2 1).

12. Installation selon la revendication 10, **caractérisée en ce qu'**elle comprend en outre :
- des moyens (7, 7a) destinés à introduire une portion majeure de l'apport de dioxyde de carbone dans ladite première unité d'extraction (21), et
- des moyens (7, 7b) destinés à introduire une portion mineure résiduelle de l'apport de dioxyde de carbone dans ledit réacteur (19) pour une synthèse d'urée.

13. Installation selon l'une quelconque des revendications précédentes 4 à 9, **caractérisée en ce que** ladite seconde unité d'extraction(22) est une unité d'extraction thermique comportant également du dioxyde de carbone en tant qu'agent d'extraction.

14. Installation selon la revendication 13, **caractérisée en ce qu'**elle comprend en outre :
- des moyens (7, 7a) destinés à introduire une portion majeure de l'apport de dioxyde de carbone dans ladite première unité d'extraction (21), et
- des moyens (7, 7b, 7c) destinés à introduire une portion mineure résiduelle de l'apport de dioxyde de carbone dans ladite seconde unité d'extraction (22) et/ou dans ledit réacteur (19) pour une synthèse d'urée.

15. Procédé selon la revendication 1, **caractérisé en ce que** ladite réaction entre de l'ammoniac et du dioxyde de carbone est mise en oeuvre dans un réacteur (19) ; ledit traitement d'extraction est mis en oeuvre dans une première unité d'extraction thermique (21) comportant également du dioxyde de carbone en tant qu'agent d'extraction, permettant de soumettre ledit mélange à un traitement de décomposition partielle du carbamate et de séparation partielle de l'ammoniac libre ; ladite condensation sensiblement totale est mise en oeuvre dans un condensateur de carbamate (20) ; ledit recyclage de ladite première portion de carbamate dans ledit réacteur (19) est mis en oeuvre au moyen d'un éjecteur (15) qui utilise de l'ammoniac liquide à titre de fluide d'entraînement ; ledit traitement de décomposition partielle de ladite au moins une partie de ladite seconde portion de carbamate en solution aqueuse est mis en oeuvre dans une seconde unité d'extraction(22) ; ladite au moins une partie dudit second flux comprenant de l'ammoniac et du dioxyde de carbone en phase vapeur est recyclée dans le réacteur (19) et/ou dans la première unité d'extraction (21).

16. Procédé selon la revendication 15, **caractérisé en ce que** le traitement de décomposition partielle de ladite au moins une partie de la seconde portion de carbamate en solution aqueuse est mis en oeuvre dans la seconde unité d'extraction (22) à une pression correspondant sensiblement à la pression existant dans le réacteur (19).

17. Procédé selon la revendication 16, **caractérisé en ce qu'**il comprend en outre l'étape consistant à :
- introduire le flux comprenant un carbamate résiduel en solution aqueuse résultant du traitement de décomposition partielle de la seconde portion de carbamate dans ladite section de récupération d'urée.

18. Procédé selon la revendication 17, **caractérisé en ce qu'**il comprend en outre les étapes consistant à :
- introduire une première portion d'ammoniac liquide dans ledit réacteur (19) pour une synthèse d'urée, et
- introduire une seconde portion d'ammoniac liquide dans ledit condensateur de carbamate (20).

19. Procédé selon la revendication 18, **caractérisé en ce que** ladite seconde unité d'extraction (22) est une unité d'extraction thermique et **en ce qu'**il comprend en outre l'étape consistant à introduire tout le dioxyde de carbone dans ladite première unité d'extraction (21).

20. Procédé selon la revendication 18, **caractérisé en ce que** ladite seconde unité d'extraction (22) est une unité d'extraction thermique et **en ce qu'**il comprend en outre les étapes consistant à :
- introduire une portion majeure de l'apport de dioxyde de carbone dans ladite première unité d'extraction (21), et
- introduire une portion mineure résiduelle de dioxyde de carbone dans ledit réacteur (19) pour une synthèse d'urée.

21. Procédé selon la revendication 18, **caractérisé en ce que** ladite seconde unité d'extraction (22) est une unité d'extraction thermique comportant également du dioxyde de carbone en tant qu'agent d'extraction et **en ce qu'**il comprend en outre les étapes consistant à :
- introduire une portion majeure de dioxyde de carbone dans ladite première mité d'extraction (21),
- introduire une portion mineure résiduelle de dioxyde de carbone dans ladite seconde unité d'extraction (22) et/ou dans ledit réacteur (19) pour une synthèse d'urée.

22. Procédé selon la revendication 20 ou la revendication 21, **caractérisé en ce que** ladite portion majeure de l'apport de dioxyde de carbone représente au moins 90 % de l'apport total de dioxyde de carbone.

23. Procédé destiné à moderniser une installation de production d'urée du type comprenant :
- un réacteur (31) pour une synthèse d'urée ;
- une première unité d'extraction thermique (32) comportant également du dioxyde de carbone en tant qu'agent d'extraction destinée a soumettre un mélange réactionnel comprenant de l'urée, un carbamate et de l'ammoniac libre en solution aqueuse sortant du réacteur (31) à un traitement de décomposition partielle de carbamate et de séparation partielle de carbamate et de séparation partielle de l'ammoniac libre, permettant d'obtenir de ce fait un premier flux comprenant de l'ammoniac et du dioxyde de carbone en phase vapeur et un flux comprenant de l'urée et un carbamate résiduel en solution aqueuse, respectivement,
- une section de récupération d'urée destinée à séparer l'urée dudit flux comprenant de l'urée et un carbamate résiduel en solution aqueuse sortant de la première unité d'extraction (32), permettant d'obtenir une seconde portion de carbamate en solution aqueuse,
- au moins une unité de condensation (33) destinée à soumettre à une condensation partielle le premier flux comprenant de l'ammoniac et du dioxyde de carbone en phase vapeur sortant de la première unité d'extraction (32), permettant d'obtenir de ce fait un flux liquide comprenant un carbamate en solution aqueuse et un flux gazeux comprenant de l'ammoniac et du dioxyde de carbone en phase vapeur,
- des moyens destinés à introduire respectivement le flux comprenant un carbamate en solution aqueuse et le flux gazeux comprenant de l'ammoniac et du dioxyde de carbone en phase vapeur dans le réacteur (31) pour une synthèse d'urée,
ledit procédé de modernisation étant **caractérisé en ce qu'**il comprend les étapes consistant à :
- transformer ladite au moins une unité de condensation (33) dans une unité de condensation totale afin de soumettre à une condensation sensiblement totale ledit premier flux comprenant de l'ammoniac et du dioxyde de carbone en phase vapeur, permettant d'obtenir un flux liquide comprenant de l'urée et un carbamate en solution aqueuse,
- produire une seconde unité d'extraction (50) destinée à soumettre au moins une partie de ladite seconde portion de carbamate en solution aqueuse à un traitement de décomposition partielle, permettant d'obtenir un second flux comprenant de l'ammoniac et du dioxyde de carbone en phase vapeur,
- produire des moyens destinés à recycler au moins une partie du second flux comprenant de l'ammoniac et du dioxyde de carbone en phase vapeur dans le réacteur (31) et/ou dans la première unité d'extraction (32)

24. Procédé selon la revendication 23, dans lequel ladite unité de condensation (33) destinée à soumettre à une condensation partielle le premier flux comprenant de l'ammoniac et du dioxyde de carbone en phase vapeur sortant de la première unité d'extraction (32) est du type film et est transformée en une unité de condensation du type submergé.

25. Procédé selon la revendication 24, **caractérisé en ce qu'**il comprend en outre :
- des moyens (54, 44, 56) destinés à introduire une première portion d'ammoniac liquide dans ledit réacteur (31) pour une synthèse d'urée, et
- un moyen (58) destiné à introduire une seconde portion d'ammoniac liquide dans ladite unité de condensation (33).

26. Procédé selon la revendication 25, **caractérisé en ce que** ladite seconde unité d'extraction (50) est une unité d'extraction thermique.

27. Procédé selon la revendication 26, **caractérisé en ce qu'**il comprend en outre des moyens destinés à introduire tout l'apport de dioxyde de carbone dans ladite première unité d'extraction (32).

28. Procédé selon la revendication 26, **caractérisé en ce qu'**il comprend en outre :
- des moyens (59) destinés à introduire une portion majeure de l'apport de dioxyde de carbone dans ladite première unité d'extraction(32), et
- des moyens (57) destinés à introduire une portion mineure résiduelle de l'apport de dioxyde de carbone dans ledit réacteur (31) pour une synthèse d'urée.

29. Procédé selon la revendication 25, **caractérisé en ce que** ladite seconde unité d'extraction (50) est une unité d'extraction thermique comportant également du dioxyde de carbone en tant qu'agent d'extraction.

30. Procédé selon la revendication 29, **caractérisé en ce qu'**il comprend en outre :
- des moyens (59) destinés à introduire une portion majeure de l'apport de dioxyde de carbone dans ladite première unité d'extraction(32), et
- des moyens destinés à introduire une portion mineure résiduelle de l'apport de dioxyde de carbone dans ladite seconde unité d'extraction (50) et/ou dans ledit réacteur (31) pour une synthèse d'urée.
